# FASCICULE DE BREVET EUROPEEN

(11) **EP 1 551 297 B1**
(45) Date de publication et mention de la délivrance du brevet: **18.06.2008**
(21) Numéro de dépôt: 03776955.1
(22) Date de dépôt: 03.10.2003
(51) Int. Cl.: A61B 5/103

(54) **DISPOSITIF ET PROCEDE DE MESURE DIRECTE DE PH ET D'ETAT D'OXYDATION**
VORRICHTUNG UND VERFAHREN ZUR DIREKTEN MESSUNG DES PH-WERTES UND DES OXIDATIONSZUSTANDES
DEVICE AND METHOD FOR DIRECT MEASUREMENT OF PH AND OXIDATION STATE

(30) Priorité: 03.10.2002 FR 0212232
(43) Date de publication de la demande: 13.07.2005
(73) Titulaire: Pierre Fabre Dermo-Cosmétique, 92100 Boulogne-Billancourt (FR)
(72) Inventeur: GROS, Pierre, 31200 Toulouse (FR); REDOULES, Daniel, F-31300 Toulouse (FR); ETCHEVERRY, Luc, F-31450 Montlaur (FR); COMTAT, Maurice, 81500 Lavaur (FR); TARROUX, Roger, F-31300 Toulouse (FR); GALL, Yvon, F-31120 Portet-sur-Garonne (FR)
(74) Mandataire: Le Forestier, Eric
(86) Numéro de dépôt international: PCT/FR2003/002908
(87) Numéro de publication internationale: WO 2004/030537

(56) Documents cités:
- WO-A-96/13193
- US-A- 4 932 410
- US-A- 5 185 922
- US-A- 5 353 802
- US-A- 5 380 422
- US-A- 5 938 903
- YOSHITAKE YAMAMOTO ET AL: "INSTANTANEOUS MEASUREMENT OF ELECTRICAL PARAMETERS IN A PALM DURINGELECTRODERMAL ACTIVITY" IEEE TRANSACTIONS ON INSTRUMENTATION AND MEASUREMENT, IEEE INC. NEW YORK, US, vol. 45, no. 2, 1 avril 1996 (1996-04-01), pages 483-486, XP000591420 ISSN: 0018-9456

## Description

### DOMAINE TECHNIQUE GENERAL.

L'invention concerne la mesure du pH et de l'état d'oxydation de la peau, à l'aide de mesures directes et localisées.

Plus précisément, elle concerne un capteur électrochimique et la mise au point d'un procédé analytique permettant de déterminer simultanément, facilement et rapidement le pH et l'état d'oxydation de la peau.

### ETAT DE L'ART.

La peau fait partie des tissus en perpétuelle interaction avec l'environnement.

Sa fonction principale est de protéger le corps humain contre les agressions extérieures d'origine chimique, microbienne et lumineuse.

Le vieillissement cutané est en grande partie lié, comme beaucoup d'autres processus dégénératifs, à l'accumulation d'agressions oxydantes.

La peau a développé toutes sortes de systèmes de défense, d'une part enzymatique comme par exemple la superoxyde dismutase (SOD), la glutathion peroxydase et la catalase, et d'autre part non enzymatique comme les vitamines E et C, l'acide urique, le glutathion.

Un certain nombre d'études rapporte une diminution des capacités des systèmes de défense contre le stress oxydatif. Par exemple, une irradiation de la couche cornée à des doses subérythémales entraîne une diminution de a-tocophérol. Des carences en vitamine C ont également été constatées comme suite à des expositions répétées à l'ozone.

Cependant, le lien entre le stress oxydatif et l'état d'oxydation de la surface cutanée reste encore à établir.

De même, il est intéressant de connaître l'évolution des pH locaux sous l'influence de ou en réponse à ce stress. Des modifications de pH peuvent en effet traduire une possible régulation des enzymes antioxydantes de la fonction barrière.

L'influence de l'environnement sur le potentiel antioxydant cutané justifie donc l'intérêt porté au développement des capteurs de mesure de pH et d'état d'oxydation à la surface de la peau.

On connaît de nombreux dispositifs et procédés de mesure de pH et d'état d'oxydation à la surface de la peau.

Généralement, le pH est déterminé par une mesure effectuée avec une électrode à membrane de verre, combinée à une électrode de référence, connue en soi par l'homme du métier.. L'électrode est de taille macroscopique, la surface caractéristique de la zone mesurée étant égale à un ou plusieurs millimètres carrés.

La mesure est effectuée en appliquant fortement l'électrode sur la peau. Pour assurer une conductivité suffisante, un film d'eau est déposé sur la peau avant la mesure.

De même, on connaît par le document WO 96/13193 un procédé et dispositif de mesure non invasive des propriétés antioxydantes de la peau.

Selon l'enseignement de ce document, on analyse l'évolution de la composition d'une solution aqueuse déposée à la surface de la peau. Au cours du temps, les molécules antioxydantes présentes dans la couche cornée diffusent dans la solution. Les mesures consistent, soit à tracer des voltammogrammes cycliques d'une solution tamponnée, soit à mesurer la variation du potentiel à courant nul d'une solution d'iode réagissant avec les molécules antioxydantes.

Les dispositifs et techniques précédents présentent cependant des inconvénients.

Les extrémités des électrodes de mesure sont de grande surface, de l'ordre du millimètre carré. Les mesures ne sont donc pas très localisées.

De plus, il y a obligation de disposer à la surface de la peau un solvant ou un gel avant d'effectuer la mesure afin d'avoir une bonne conductivité. L'application du produit est une étape supplémentaire dans l'obtention des résultats, et peut également fausser les mesures.

En outre, le fait de devoir appuyer fortement sur l'électrode provoque des désagréments pour le patient ou la personne qui effectue les mesures. La pression exercée modifie également la surface cutanée analysée.

Le document US-A-538 422 concerne un dispositif de mesure des propriétés physico-chimiques de la peau comportant des moyens de génération, de régulation et de traitement de tensions électriques, une électrode de travail, une électrode de référence et une électrode auxiliaire.

Le document US-A-4 932 410 décrit un procédé de mesure des propriétés physico-chimiques de la peau, notamment son pH et son état d'oxydation, a l'aide d'un dispositif comprenant les caractéristiques du préambule de la revendication 8.

Enfin, les mesures de pH et d'état d'oxydation simultanées de la peau ne sont pas possibles avec les dispositifs de l'art antérieur. On doit effectuer successivement les mesures avec des appareils différents. On ne peut donc pas avoir une bonne corrélation, ni temporelle ni spatiale, entre les résultats des mesures de pH et des mesures d'état d'oxydation de la peau.

### PRESENTATION DE L'INVENTION.

L'invention propose de pallier ces inconvénients.

Un premier but de l'invention est de proposer un capteur électrochimique et un procédé de mesure permettant des mesures directes, localisées et simultanées du pH et de l'état d'oxydation de la peau, afin d'obtenir une bonne corrélation entre les différents résultats des mesures.

Un autre but de l'invention est de proposer un dispositif de mesure dont les électrodes de mesure soient de plus petite taille.

Un autre but de l'invention est de proposer un dispositif de mesure qui permette une mesure directe sur la peau, sans addition de solvant ni de gel.

Un but de l'invention est de proposer un dispositif dont la surface cutanée analysée ne varie pas pendant la mesure.

Enfin, un autre but de l'invention est de proposer un dispositif de mesure plus précis.

A cet effet, l'invention propose un dispositif de mesure des propriétés physico-chimiques de la peau, notamment son pH et son état d'oxydation, comme défini dans la rev.1.

L'invention est avantageusement complétée par les caractéristiques suivantes, prises seules ou en une quelconque de leur combinaison techniquement possible :
- l'électrode de travail comporte un tube capillaire isolant électrique à l'intérieur duquel s'étend un fil métallique dont l'extrémité libre forme un disque apte à être mis en contact direct avec la peau lors des mesures ;
- le fil de l'électrode de travail est en platine non modifié ;
- le diamètre extérieur moyen de l'extrémité libre de l'électrode de travail est sensiblement égal à 120µm ;
- l'électrode auxiliaire est en platine, en or,en carbone, en tungstène ou en bismuth non modifiés ;
- l'électrode de travail et l'électrode auxiliaire comprennent des fils étant recouverts d'un isolant électrique ;
- le fil de l'électrode de travail s'étend dans un premier tube capillaire isolant électrique, l'électrode auxiliaire s'étendant dans un deuxième tube capillaire isolant électrique extérieur et concentrique au premier, une partie de l'électrode auxiliaire étant formée d'une feuille entourant le premier tube tout en s'étendant dans le second.

L'invention concerne également le procédé de fabrication des électrodes d'un tel dispositif, ainsi que le procédé d'utilisation d'un tel dispositif.

### PRESENTATION DES FIGURES

D'autres caractéristiques, buts et avantages de l'invention ressortiront de la description qui suit, qui est purement illustrative et non limitative et qui doit être lue en regard des dessins annexés sur lesquels :
- la figure 1 est une représentation schématique d'un montage possible d'un dispositif de mesure selon l'invention ;
- la figure 2 est une représentation schématique d'une coupe longitudinale d'un mode de réalisation possible de microélectrode de travail ;
- la figure 3 est une représentation schématique d'une coupe radiale d'un mode de réalisation de la figure 2 ;
- la figure 4 est une représentation schématique d'une première variante possible de mode de réalisation des électrodes d'un dispositif de mesure selon l'invention ;
- la figure 5 est une représentation schématique d'une deuxième variante possible de mode de réalisation des électrodes d'un dispositif de mesure selon l'invention ;
- la figure 6 est l'évolution du potentiel à courant nul du microcapteur appliqué sur la peau de l'avant-bras ;
- la figure 7 est le voltammogramme cyclique obtenu avec le microcapteur appliqué sur la peau de l'avant-bras ; et
- la figure 8 montre la détermination du pH à la surface de la peau par la mesure du potentiel à courant nul du micromicrocapteur.

### DESCRIPTION DETAILLEE DE L'INVENTION.

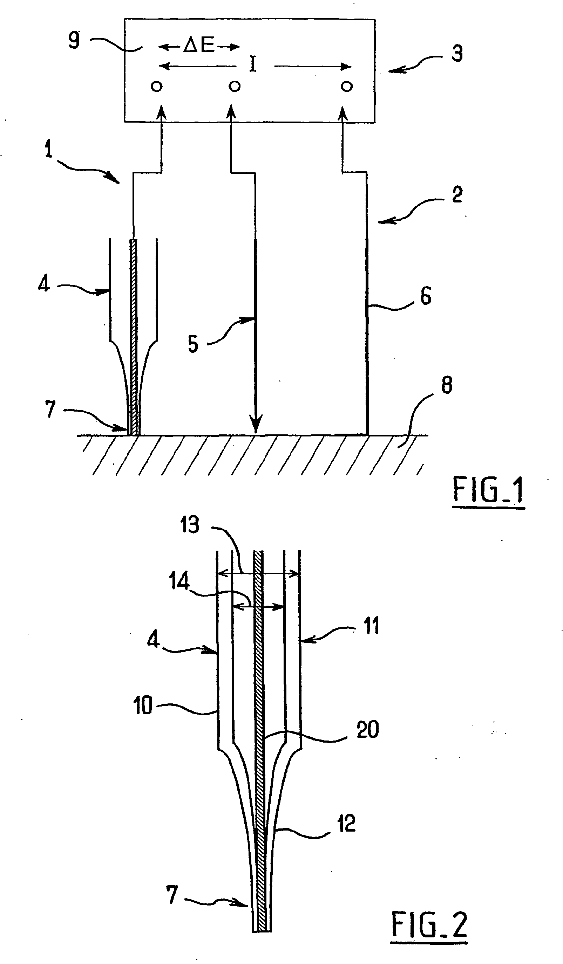

La figure 1 est une représentation schématique d'un montage possible d'un dispositif 1 de mesure.

Le dispositif 1 comporte principalement deux parties : une partie 2 est apte à effectuer les mesures sur la peau ; une partie 3 comprend un potentiostat 9 apte à générer et à réguler des tensions électriques vers la partie 2, et un dispositif d'acquisition et de traitement des résultats des mesures reçues de la partie 2.

La partie 2 de mesure, également appelée « capteur » dans la suite de la présente description, comporte trois éléments principaux.

Le premier élément est une microélectrode métallique 4 utilisée comme électrode de travail. Elle est préférentiellement en platine. La microélectrode 4 comporte une extrémité libre 7.

Le deuxième élément est un fil 6 utilisé comme électrode auxiliaire. Préférentiellement, le fil 6 est en platine, en or,en carbone, en tungstène ou en bismuth.

Enfin, le troisième élément est une électrode de référence 5 au chlorure d'argent Ag / AgCl⁻ / Cl⁻ₛₐₜ, par rapport à laquelle tous les potentiels sont exprimés. Une telle électrode de référence est connue en soi de l'homme du métier.

Les trois électrodes 4, 5 et 6 sont reliées à la partie 3. La partie 3 comporte un potentiostat 9 et un dispositif d'acquisition et de traitement des résultats des mesures

Le potentiostat 9 est apte à appliquer et à réguler une différence de potentiel ΔE (notée également E (V) % Ag/AgCl/Clₛₐₜ) variable entre l'électrode de travail 4 et l'électrode de référence 5. Le dispositif d'acquisition et de traitement des résultats est apte à mesurer le courant 1 traversant le circuit électrique comportant l'électrode de travail 4 et l'électrode auxiliaire 6.

Lorsque l'on veut effectuer une mesure de pH et d'état d'oxydation, l'extrémité 7 de la microélectrode 4 est positionnée à la surface de la peau 8. Le positionnement de l'électrode 4 est effectué préférentiellement à l'aide d'un micromanipulateur de précision non représenté sur la figure 1.

L'électrode auxiliaire 6 et l'électrode de référence 5 sont également disposées sur la peau 8 à proximité de l'extrémité 7. On prendra de préférence une distance entre les électrodes 4, 5 et 6 inférieure ou égale à 5 cm.

Lors d'une mesure, deux types d'expériences sont réalisés successivement.

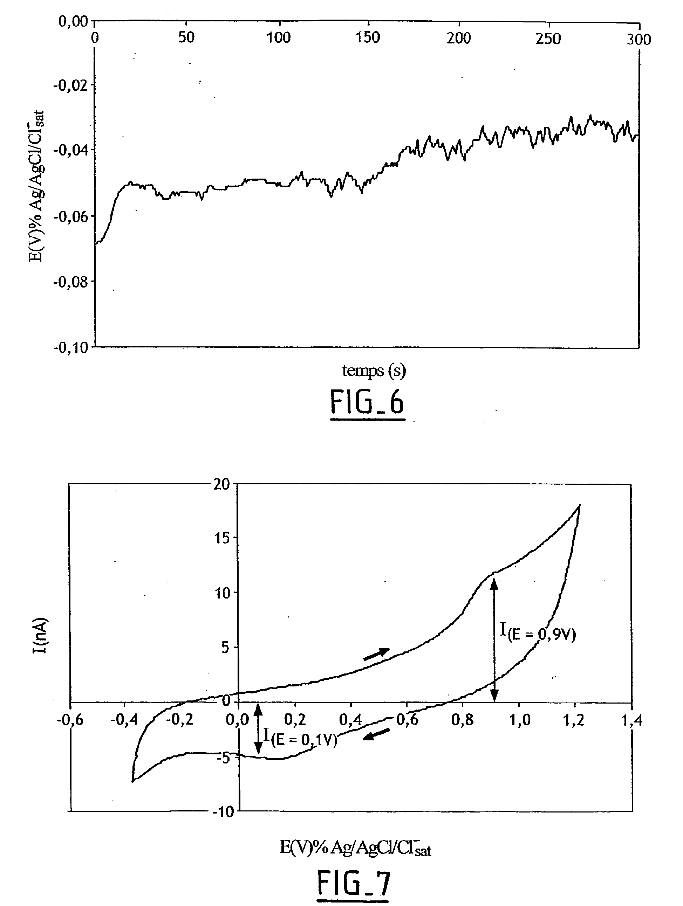

Le premier type de mesure réside dans la mesure de la différence de potentiel ΔE à courant I nul entre l'électrode de travail 4 et l'électrode de référence 5. Un exemple de courbe obtenue par cette mesure est visible à la figure 6. Ce type de mesure a une utilité dans la mesure du pH et de l'état d'oxydation de la peau.

Le deuxième type de mesure consiste à enregistrer un voltammogramme cyclique de l'électrode 4 pour des potentiels compris entre -0,4V et 1,2V, à la vitesse de balayage de 50 mV/s.

La courbe ainsi obtenue enregistre le courant I d'électrolyse traversant le circuit constitué de l'électrode de travail 4 et l'électrode auxiliaire 6, en fonction de la différence de potentiel ΔE appliquée entre l'électrode de travail 4 et l'électrode de référence 5. Un exemple de voltammogramme cyclique est visible à la figure 7. Sur cette figure, on note la présence de flèches qui indiquent le sens d'obtention de la courbe lors du balayage à partir du potentiel -0,4V vers le potentiel 1,2V (partie haute de la courbe, trajet correspondant à l'oxydation) puis de 1,2V vers -0,4V (partie basse de la courbe, trajet correspondant à la réduction).

Sur cette courbe, on note la présence de deux plateaux d'intensité. Le premier plateau est situé sur la partie haute de la courbe, entre les potentiels compris entre 0,8V et 1V. Ce plateau a une utilité pour l'évaluation de l'état d'oxydation. Le deuxième plateau d'intensité est situé entre les potentiels 0V et 0,2V sur la partie basse de la courbe. Ce plateau a une utilité pour l'obtention du pH de la peau.

Le protocole opératoire repose sur les manipulations successives suivantes.

On mesure d'abord la différence de potentiel à courant nul entre l'électrode de travail 4 et l'électrode de référence, afin d'obtenir la courbe selon la figure 6. Cette mesure est effectuée durant 150 secondes à 300 secondes. On trace ensuite le voltammogramme cyclique entre les potentiels -0,4V et 1,2V comme celui représenté à la figure 7.

On déduit ensuite les valeurs du pH de la peau et de l'état d'oxydation de ces deux figures.

D'une part, la détermination du pH découle de la mesure de la différence de potentiel ΔE à courant 1 nul entre l'électrode de travail 4 et l'électrode de référence 5.

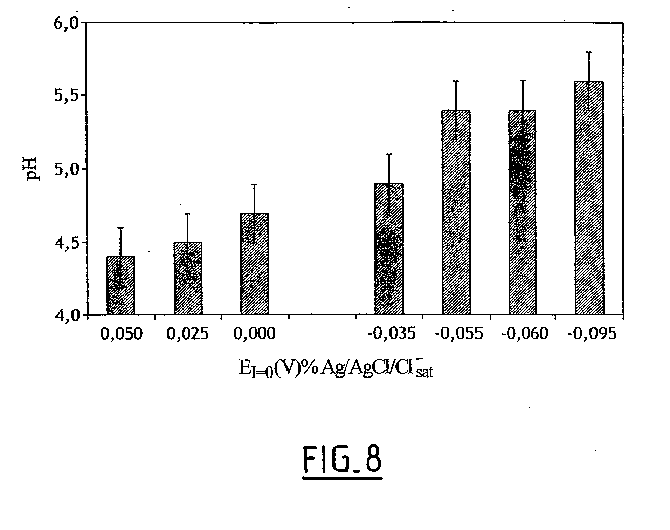

En effet, le graphe de la figure 8 est établi lors d'un calibrage préalable du dispositif de mesure. On construit cette courbe en corrélant la différence de potentiel ΔE à courant nul mesurée sur la peau de différents sujets, au pH de la peau mesuré par une électrode à membrane de verre connue en soi par l'homme de métier. Cette courbe est construite avec un grand nombre de potentiels en abscisse, auxquels correspondent des pH mesurés. Elle est ensuite mémorisée par des moyens compris dans le dispositif d'acquisition et de traitement des résultats des mesures.

Ainsi, lors d'une utilisation postérieure de l'appareil, c'est-à-dire lors d'une mesure, l'appareil effectue la mesure de la différence de potentiel ΔE à courant nul sur la peau afin d'obtenir la courbe selon la figure 6 (sur la figure 6, cette valeur est à peu près égale à -0,04V)

A partir de la courbe de correspondance selon la figure 8, l'appareil donne à l'utilisateur la valeur du pH de la peau.

Par ailleurs, la détermination du pH peut aussi être effectuée à partir du tracé du voltammogramme cyclique de la figure 7. La valeur du pH est déduite de l'intensité du courant d'électrolyse lorsque le potentiel de l'électrode de travail en platine est compris entre -0,2V et 0,2V, et préférentiellement égal à 0,1 V. On utilise donc le deuxième plateau d'intensité de la courbe obtenue à la figure 7.

Le principe de détermination du pH est le même que précédemment. On calibre l'appareil en traçant les voltammogrammes cycliques obtenus sur la peau de différents sujets et on corrèle l'intensité du courant d'électrolyse, lorsque le potentiel de l'électrode de travail est égal à 0,1V, au pH de la peau mesuré par une électrode à membrane de verre connue de l'homme du métier. On construit alors un tableau ou une courbe de correspondance entre les intensités mesurées sur la peau et les valeurs de pH, cette relation de correspondance étant entrée dans le dispositif d'acquisition et de traitement des résultats des mesures. La relation de correspondance n'est pas montrée dans les présentes figures.

Ainsi, après l'obtention de la courbe de la figure 7 lors d'une mesure, l'appareil est capable de donner la valeur du pH grâce à cette correspondance.

Des exemples de mesures sont indiqués dans le tableau 1 ci-dessous. Ce dernier représente la détermination du pH de la peau de quatre sujets sur lesquels sont effectuées des mesures. Les troisième et quatrième colonnes du tableau 1 montrent les résultats expérimentaux à partir desquels on déduit la valeur du pH de la deuxième colonne. Pour la quatrième colonne, on utilise le deuxième plateau de la courbe de la figure 7.

**Tableau 1.**

| **Sujet** | **pH** | **EI₍₁₌₀₎ (V)** | **I_{(E=0.1V)} (nA)** |
|---|---|---|---|
| 1 | 4.9 | -0.035 | -1.8 |
| 2 | 5.4 | -0.06 | -4.9 |
| 3 | 5.4 | -0.055 | -5.5 |
| 4 | 5.6 | -0.095 | -5.3 |

Le fait que le pH puisse être déterminé selon deux méthodes permet d'affiner les mesures et de les vérifier.

D'autre part, l'évaluation de l'état d'oxydation de la peau est issue des deux mesures complémentaires entre elles suivantes.

En premier lieu, on utilise la mesure de la différence de potentiel à courant nul entre l'électrode de travail 4 en platine et l'électrode de référence 5. Il s'agit de la valeur obtenue à partir de la courbe de la figure 6.

En second lieu, on utilise la mesure de l'intensité du courant d'électrolyse du voltammogramme cyclique (figure 7) lorsque le potentiel de l'électrode de travail 4 est compris entre 0,8V et 1,0V, et de préférence égal à 0,9V. On utilise donc le premier plateau d'intensité. Le tableau 2 suivant montre une série de mesures complémentaires, des mesures par E à I = 0 et des mesures pour I à E = 0,9V.

**Tableau 2.**

| **Sujet** | **E_{(I=0)} (V)** | **I_{(E=0.9V)} (nA)** |
|---|---|---|
| **1** | -0.035 | 3.0 |
| **2** | -0.055 | 8.9 |
| **3** | -0.06 | 8.9 |
| **4** | -0.095 | 9 |

Une surface de peau oxydée est caractérisée par une valeur élevée du potentiel à courant nul et par une faible intensité du courant d'électrolyse. A l'inverse, une surface de peau peu oxydée est caractérisée par une valeur basse du potentiel à courant nul et par une forte intensité du courant d'électrolyse.

Ainsi dans le tableau 2, la peau du sujet 1 est relativement oxydée, alors que la peau du sujet 4 est moins oxydée.

Les résultats donnés par l'appareil permettent donc de déterminer l'état d'oxydation de la peau du sujet.

Comme on l'a vu, les deux séries de mesures sont complémentaires et permettent d'affiner les mesures et de les vérifier.

Le procédé analytique permet d'effectuer directement des mesures à la surface de la peau, sans addition de solvant ni de gel, et sans modification de surface du capteur.

On va maintenant décrire plus en détails les électrodes de la partie 2 du dispositif 1.

La figure 2 est une représentation schématique d'une coupe longitudinale d'un mode de réalisation possible de microélectrode de travail 4.

Sur la figure 2, la microélectrode 4 comporte un capillaire 10 isolant, préférentiellement en verre, dans lequel s'étend un fil de platine 20.

Le capillaire 10 comporte une première partie 11 reliée à la partie 3 du dispositif, une autre partie 12 s'étendant à partir de la partie 11 vers l'extrémité libre 7 de l'électrode 4.

La partie 11 forme un tube à symétrie de révolution circulaire. Le diamètre externe 13 de la partie 11 est sensiblement égal à 1,2mm par exemple. Le diamètre interne 14 est sensiblement égal à 0,7mm par exemple.

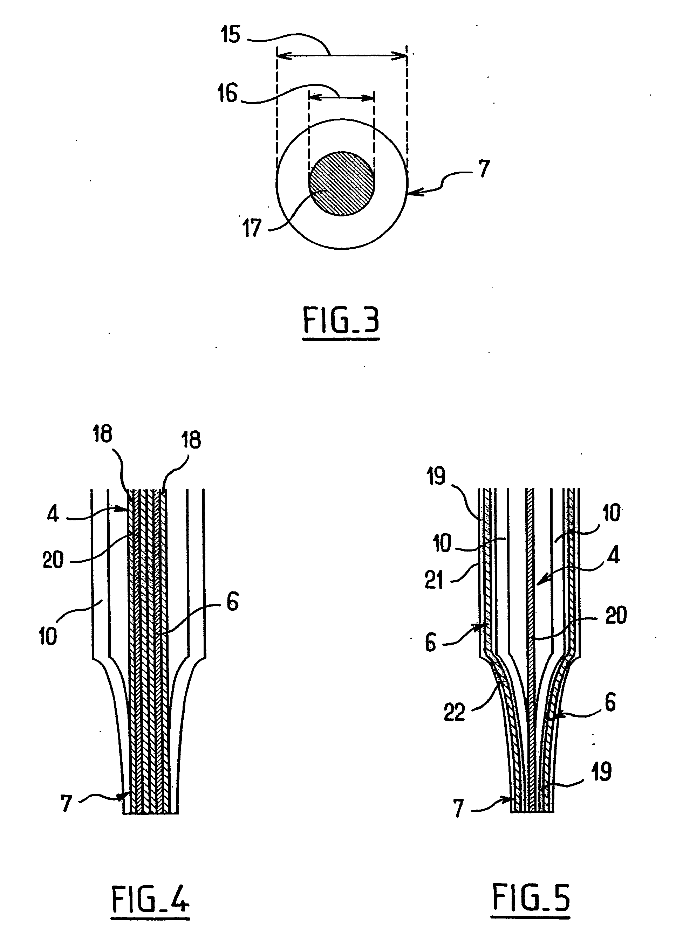

La partie 12 est également à symétrie de révolution, mais de section droite de plus en plus faible, à partir de la partie 11 vers l'extrémité libre 7. La figure 3 montre, que le diamètre externe 15 de l'extrémité 7 est de l'ordre de 120µm environ. Le diamètre externe du fil de platine 20 à l'extrémité 7 est de l'ordre de 50µm.

Le capteur électrochimique permet de déterminer simultanément le pH et l'état d'oxydation sur une surface de peau inférieure à 10⁻² mm², pour un temps d'analyse inférieur à 5 minutes. Il assure ainsi une très bonne résolution analytique dans l'espace et dans le temps.

La composition du fil 20 est préférentiellement Pt 90% - Ir 10%, garantissant ainsi une bonne résistance mécanique.

Lors de la fabrication de l'électrode 4, le fil 20 est introduit dans le tube 10 à section cylindrique de révolution. Le fil 20 est ensuite étiré à l'aide d'une microétireuse. L'extrémité 7 de l'électrode 4 est alors chauffée de façon à parfaitement enrober le fil de platine de verre, ce qui donne la forme de la partie 12 de l'électrode 4.

L'extrémité 7 est ensuite poncée sur une résine diamantée de façon à obtenir un disque 17 de platine plan, le disque étant visible à la figure 3.

Lors du nettoyage de l'électrode 4 entre deux séries de mesures, la surface du disque 17 de platine situé à l'extrémité 7 est régénérée en faisant subir à la microélectrode 4 des cycles répétés de potentiel entre -0,2V et 1,2V à la vitesse de balayage de 50 mV/s, dans une solution d'acide sulfurique à 0,5 mol L⁻¹.

Les figures 4 et 5 représentent schématiquement des variantes de mode de réalisation des électrodes 4 et 6 de la partie 2 du dispositif 1.

Les variantes des figures 4 et 5 représentent des modes de réalisation préférés.

En effet, dans les modes de réalisation des figures 4 et 5, la même électrode ou capillaire comporte l'électrode de travail 4 et l'électrode auxiliaire 6.

La figure 4 montre un mode de réalisation selon lequel le fil métallique 20 de l'électrode 4 et le fil 6 de l'électrode auxiliaire sont introduits dans le capillaire 10 de verre. Les deux fils sont cependant recouverts chacun d'un isolant électrique 18 qui permet des les séparer électriquement l'un de l'autre, bien qu'ils soient spatialement très proches.

Dans le mode de réalisation représenté à la figure 5, l'électrode comporte deux capillaires concentriques. Un premier capillaire 10 comporte le fil 20 de l'électrode de travail 4. Un deuxième capillaire 19 est concentrique au capillaire 10 tout en le recouvrant. Le capillaire 19 comporte ainsi au moins une paroi externe 21 parallèle aux parois du capillaire 10.

Ainsi, le capillaire 10 comporte le fil métallique 20 de l'électrode de travail 4 tandis que le capillaire 19 comporte une feuille 22 de l'électrode auxiliaire 6. La feuille 22 est enroulée autour du capillaire 10, tout en s'étendant dans le capillaire 19.

Les modes de fabrication de telles variantes, notamment de leur extrémité libre 7, sont identiques à celui décrit plus haut pour la fabrication de l'électrode 4 de la figure 2.

On obtient avec les variantes des figures 4 et 5 les mêmes courbes de résultats que celles représentées aux figures 6 et 7.

Les dimensions du capteur permettent ainsi d'analyser une surface de peau de l'ordre de 10⁻² mm².

## Revendications

1. Dispositif (1) de mesure des propriétés physico-chimiques de la peau (8), notamment son pH et son état d'oxydation, comportant des moyens (9) de génération, de régulation et de traitement de tensions électriques reliés à des moyens (2) de mesure de propriétés de la peau comportant une électrode de travail (4), l'électrode de travail (4) comportant un tube capillaire (10) isolant électrique à l'intérieur duquel s'étend un fil métallique (20) dont l'extrémité libre (7) forme un disque (17) apte à être mis en contact direct avec la peau lors des mesures, les moyens de mesure comportant en outre une électrode de référence (5) et une électrode auxiliaire (6) aptes à être mises elles aussi en contact direct avec la peau lors des mesures, **caractérisé en ce que** l'électrode auxiliaire (6) et l'électrode de travail (4) sont situées dans un tube capillaire commun.

2. Dispositif selon la revendication 1, **caractérisé en ce que** le fil (20) de l'électrode de travail (4) est en platine non modifié.

3. Dispositif selon l'une des revendications 1 ou 2, **caractérisé en ce que** le diamètre extérieur moyen (15) de l'extrémité libre (7) de l'électrode de travail (4) est sensiblement égal à 120µm.

4. Dispositif selon l'une des revendications 1 à 3, **caractérisé en ce que** l'électrode auxiliaire (6) est en platine, en or, en carbone, en tungstène ou en bismuth non modifiés.

5. Dispositif selon l'une des revendications 1 à 4, **caractérisé en ce que** l'électrode de travail (4) et l'électrode auxiliaire (6) comprennent un fil, les fils desdites électrodes étant recouverts d'un isolant électrique (18).

6. Dispositif selon l'une des revendications 1 à 4, **caractérisé en ce que** le fil métallique (20) de l'électrode de travail (4) s'étend dans un premier tube capillaire (10) isolant électrique, l'électrode auxiliaire (6) s'étendant dans un deuxième tube capillaire (19) isolant électrique extérieur et concentrique au premier (10), une partie de l'électrode auxiliaire (6) étant formée d'une feuille (22) entourant le premier tube (10) tout en s'étendant dans le second.

7. Procédé de mesure des propriétés physico-chimiques de la peau, notamment son pH et son état d'oxydation, à l'aide d'un dispositif comportant des moyens (9) de génération, de régulation et de traitement de tensions électriques reliés à des moyens (2) comportant une électrode de travail (4), une électrode auxiliaire (6) et une électrode de référence (5) aptes à être mises en contact direct avec la peau, **caractérisé en ce qu'**on détermine les valeurs de pH et d'état d'oxydation en fonction de résultats obtenus par des étapes selon lesquelles :
- on mesure d'abord la différence de potentiel (ΔE) à courant (I) nul entre l'électrode de travail (4) et l'électrode de référence (5).
- on trace ensuite le voltammogramme cyclique entre les potentiels - 0,4V et 1,2V, afin d'obtenir la courbe du courant (I) d'électrolyse traversant le circuit constitué de l'électrode de travail (4) et l'électrode auxiliaire (6), en fonction de la différence de potentiel (ΔE) appliquée entre l'électrode de travail (4) et l'électrode de référence (5).

8. Procédé selon la revendication 7, **caractérisé en ce qu'**on détermine la valeur du pH à partir de la différence de potentiel à courant nul mesurée entre l'électrode de travail (4) et l'électrode de référence (5) et/ou à partir de l'intensité du courant d'électrolyse pour une valeur de la différence de potentiel appliquée entre l'électrode de travail (4) et l'électrode de référence (5) égale à 0,1 V.

9. Procédé selon l'une des revendications 7 ou 8, **caractérisé en ce qu'**on détermine l'état d'oxydation à partir de la différence de potentiel à courant nul mesurée entre l'électrode de travail (4) et l'électrode de référence (5) et/ou à partir de l'intensité du courant d'électrolyse pour une valeur de la différence de potentiel appliquée entre l'électrode de travail (4) et l'électrode de référence (5) égale à 0,9V.

## Claims

1. A device (1) for measuring physico-chemical properties of the skin (8), notably its pH and its oxidation state, including means (9) for generating, regulating and processing electrical voltages, connected to means (2) for measuring properties of the skin including a working electrode (4), the working electrode (4) including an electrically insulating capillary tube (10) inside which extends a metal wire (20), the free end of which (7) forms a disc (17) capable of being put into direct contact with the skin during measurements, the measurement means further including a reference electrode (5) and an auxiliary electrode (6) capable of being themselves also put into direct contact with the skin during measurements, **characterized in that** the auxiliary electrode (6) and the working electrode (4) are located in a common capillary tube.

2. The device according to claim 1, **characterized in that** the wire (20) of the working electrode (4) is in non-modified platinum.

3. The device according to claim 1 or 2, **characterized in that** the average outer diameter (15) of the free end (7) of the working electrode (4) is substantially equal to 120 µm.

4. The device according to any of claims 1 to 3, **characterized in that** the auxiliary electrode (6) is in non-modified platinum, gold, carbon, tungsten or bismuth.

5. The device according to any of claims 1 to 4, **characterized in that** the working electrode (4) and the auxiliary electrode (6) comprise a wire, the wires of said electrodes being covered with an electric insulator (18).

6. The device according to any of claims 1 to 4, **characterized in that** the metal wire (20) of the working electrode (4) extends in a first electrically insulating capillary tube (10), the auxiliary electrode (6) extending in a second outer electrically insulating capillary tube (19) concentrically with the first (10), a portion of the auxiliary electrode (6) being formed with a foil (22) surrounding the first tube (10) while extending into the second one.

7. A method for measuring physico-chemical properties of the skin, notably its pH and its oxidation state, by means of a device including means (9) for generating, regulating and processing electrical voltages, connected to means (2) including a working electrode (4), an auxiliary electrode (6) and a reference electrode (5) capable of being put into direct contact with the skin, **characterized in that** the pH and oxidation state values are determined according to results obtained by steps according to which:
- the potential difference (ΔE) at zero current (I) is first measured between the working electrode (4) and the reference electrode (5);
- the cyclic voltammogram between the potentials 0.4V and 1.2V is then plotted in order to obtain the curve of the electrolysis current (I) passing through the circuit consisting of the working electrode (4) and the auxiliary electrode (6), versus the potential difference (ΔE) applied between the working electrode (4) and the reference electrode (5).

8. The method according to claim 7, **characterized in that** the pH value is determined from the potential difference at zero current, measured between the working electrode (4) and the reference electrode (5) and/or from the intensity of the electrolysis current for a value of the potential difference applied between the working electrode (4) and the reference electrode (5) equal to 0.1V.

9. The method according to any of claims 7 or 8, **characterized in that** the oxidation state is determined from the potential difference at zero current, measured between the working electrode (4) and the reference electrode (5) and/or from the intensity of the electrolysis current for a value of the potential difference applied between the working electrode (4) and the reference electrode (5) equal to 0.9V.

## Patentansprüche

1. Vorrichtung (1) zum Messen von physikochemischen Eigenschaften der Haut (8), insbesondere ihres pH und ihres Oxidationszustands, umfassend Mittel (9) zur Erzeugung, Regulierung von und Behandlung mit elektrischen Spannungen, die mit Mitteln (2) zum Messen der Eigenschaften der Haut verbunden sind, welche eine Arbeitselektrode (4) umfassen, wobei die Arbeitselektrode (4) ein elektrisch isolierendes Kapillarrohr (10) umfasst, in dessen Innerem sich ein Metallfaden (20) erstreckt, dessen freies Ende (7) eine Scheibe (17) bildet, die geeignet ist, während Messungen in direkten Kontakt mit der Haut gebracht zu werden, wobei die Mittel zum Messen darüber hinaus eine Bezugselektrode (5) und eine Hilfselektrode (6) umfassen, die geeignet sind, während Messungen ebenfalls in direkten Kontakt mit der Haut gebracht zu werden, **dadurch gekennzeichnet, dass** die Hllfselektrode (6) und die Arbeitselektrode (4) sich in einem gemeinsamen Kapillarrohr befinden.

2. Vorrichtung nach Anspruch 1, **dadurch gekennzeichnet, dass** der Faden (20) der Arbeitselektrode (4) aus unmodifiziertem Platin besteht.

3. Vorrichtung nach einem der Ansprüche 1 oder 2, **dadurch gekennzeichnet, dass** der mittlere äußere Durchmesser (15) des freien Endes (7) der Arbeitselektrode (4) etwa gleich 120 µm ist.

4. Vorrichtung nach einem der Ansprüche 1 bis 3, **dadurch gekennzeichnet, dass** die Hilfselektrode (6) aus unmodifiziertem Platin, Gold, Kohlenstoff, Wolfram oder Bismut besteht.

5. Vorrichtung nach einem der Ansprüche 1 bis 4, **dadurch gekennzeichnet, dass** die Arbeitselektrode (4) und die Hilfselektrode (6) einen Faden umfassen, wobei die Fäden der Elektroden mit einem elektrischen Isolator (18) überzogen sind.

6. Vorrichtung nach einem der Ansprüche 1 bis 4, **dadurch gekennzeichnet, dass** sich der Metallfaden (20) der Arbeitselektrode (4) in einem ersten elektrisch isolierenden Kapillarrohr (10) erstreckt, wobei sich die Hilfselektrode (6) in einem zweiten elektrisch isolierenden Kapillarrohr (19) erstreckt, das außerhalb von und konzentrisch mit dem ersten (10) angeordnet ist, wobei ein Teil der Hilfselektrode (6) aus einem Blatt (22) gebildet ist, welches das erste Rohr (10) umgibt, während es sich gleichzeitig in dem zweiten erstreckt.

7. Verfahren zur Messung von physikochemischen Eigenschaften der Haut, insbesondere ihres pH und ihres Oxidationszustands, mit Hilfe einer Vorrichtung, umfassend Mittel (9) zum Erzeugen, Regulieren von und Behandlung mit elektrischen Spannungen, die mit Mitteln (2) verbunden sind, welche eine Arbeitselektrode (4), eine Hilfselektrode (6) und eine Bezugselektrode (5) umfassen, die geeignet sind, in direkten Kontakt mit der Haut gebracht zu werden, **dadurch gekennzeichnet, dass** man die Werte des pH und des Oxidationszustands als Funktion der Ergebnisse bestimmt, die durch Schritte erhalten werden, gemäß welchen:
- man zuerst den Potentialunterschied (ΔE) bei null Strom (I) zwischen der Arbeitselektrode (4) und der Bezugselektrode (5) misst;
- man anschließend die zyklische voltammetrische Kurve zwischen den Potentialen -0,4 V und 1,2 V aufzeichnet, um die Kurve des Elektrolysestroms (I), welcher den Kreislauf durchquert, der aus der Arbeitselektrode (4) und der Hilfselektrode (6) besteht, als Funktion des Potentialunterschieds (ΔE), der zwischen der Arbeitselektrode (4) und der Bezugselektrode (5) angelegt wird, zu erhalten.

8. Verfahren nach Anspruch 7, **dadurch gekennzeichnet, dass** man den pH-Wert aus dem Potentialunterschied bei null Strom, gemessen zwischen der Arbeitselektrode (4) und der Bezugselektrode (5), und/oder aus der Stärke des Elektrolysestroms bei einem Wert des Potentialunterschieds, der zwischen der Arbeitselektrode (4) und der Bezugselektrode (5) angelegt wird, gleich 0,1 V bestimmt.

9. Verfahren nach einem der Ansprüche 7 oder 8, **dadurch gekennzeichnet, dass** man den Oxidationszustand aus dem Potentialunterschied bei null Strom, gemessen zwischen der Arbeitselektrode (4) und der Bezugselektrode (5), und/oder aus der Stärke des Elektrolysestroms bei einem Wert des Potentialunterschieds, der zwischen der Arbeitselektrode (4) und der Bezugselektrode (5) angelegt wird, gleich 0,9 V bestimmt.
